# EUROPÄISCHE PATENTANMELDUNG

(11) **EP 2 213 278 A1**
(43) Veröffentlichungstag der Anmeldung: **04.08.2010**
(21) Anmeldenummer: 09001153.7
(22) Anmeldetag: 28.01.2009
(51) Int. Cl.: A61K 9/00, A61K 9/70

(54) **Pharmazeutische Zubereitung mit verbesserter Stabilität des Wirkstoffs**

(71) Anmelder: Labtec GmbH, 40764 Langenfeld (DE)
(72) Erfinder: Schwier, Nina Dr., 45219 Essen (DE); Klaffenbach, Peter Dr., 40699 Erkrath (DE); Breitenbach, Armin Dr., 51371 Leverkusen (DE)
(74) Vertreter: Von Renesse, Dorothea

(57) **Zusammenfassung**

Pharmazeutische Zubereitung, insbesondere eine filmförmige Zubereitung, enthaltend wenigstens einen pharmazeutischen Wirkstoff oder dessen Salz, wobei die Zubereitung mindestens einen Komplexbildner zur Stabilisierung des Wirkstoffs oder dessen Salzes sowie mindestens oder mehrere Polymere ausgewählt aus der Gruppe der natürlichen oder halb-synthetischen oder synthetischen hydrophilen Polymere, enthält, und der Anteil der natürlichen und halb-synthetischen Polymere größer ist als der Anteil der synthetischen Polymere.

## Beschreibung

Die Erfindung betrifft wirkstoffhaltige pharmazeutische Zubereitungen, insbesondere filmförmige Zubereitungen, die sich durch eine verbesserte chemische Stabilität des Wirkstoffs auszeichnen. Ferner betrifft die Erfindung ein Verfahren zur Herstellung dieser Zubereitungen.

Pharmazeutische Zubereitungen, insbesondere wirkstoffhaltige Filme, weisen üblicherweise eine Matrix enthaltend mindestens ein Polymer auf. Diese Polymermatrix umfasst mindestens einen Wirkstoff, z. B. einen Arzneistoff oder dessen Salz, in gelöster oder dispergierter Form. Häufig enthält die Matrix verschiedenartige Hilfsstoffe oder Zusatzstoffe , beispielsweise zur Einstellung verschiedener physikalischer, physikalisch-chemischer oder biopharmazeutischer Eigenschaften. Derartige filmförmige Zubereitungen setzen die darin enthaltenen Wirkstoffe meist am Applikationsort, beispielsweise in der Mundhöhle, frei. Diese können dann zum Beispiel bei oraler Gabe der Filme bereits in der Mundhöhle (bukkale Resorption) oder erst nach Herrunterschlucken intestinal resorbiert werden.

Viele pharmazeutische Wirkstoffe sind jedoch chemisch instabil, d.h. sie zersetzen sich bereits vor der Entfaltung ihrer eigentlichen pharmazeutischen Wirkung beispielsweise während einer längeren Lagerung oder unter Stressbedingungen. Damit verringert sich nicht nur die anfängliche Wirkstoffmenge, sondern die Abbauprodukte können auch toxisch sein und/oder zu ungewünschten Nebenwirkungen führen.

Für diese Abbaureaktionen können externe Faktoren wie beispielsweise eine Temperatur- oder Lichteinwirkung verantwortlich sein. Sie hängen darüber hinaus jedoch auch von der chemischen Konstitution der pharmazeutischen Zusammensetzung sowie des Wirkstoffs oder dessen Salz ab.

Wesentlich sind dabei neben reduktiven Vorgängen vor allem auch oxidative Reaktionen. Diese treten besonders häufig in Gegenwart von aktivem Sauerstoff, z. B. in Gegenwart von Peroxiden, auf. Dabei handelt es sich um Autoxidationsreaktionen, die als Kettenreaktionen ablaufen. Weiterhin können auch andere radikalische Reaktionen mit vergleichbar negativem Ergebnis stattfinden. Derartige Autoxidationsprozesse und die zugrundeliegenden Reaktionsmechanismen sind dem Fachmann grundsätzlich bekannt.

Bei filmförmigen Zubereitungen ist im besonderen Maße mit diesen unerwünschten oxidativen Prozessen zu rechnen, da diese eine relative grosse Oberfläche aufweisen, die z.B. dem Angriff des Luftsauerstoffs ausgesetzt sein kann.

Um diese Abbaureaktionen zu unterdrücken, werden filmförmige Arzneiformen häufig unter Sauerstoffausschluss, z. B. unter einer Stickstoffatmosphäre, hergestellt und verpackt, oder es werden der pharmazeutischen Zusammensetzung Antioxidantien hinzugefügt.

Es hat sich aber gezeigt, dass diese Maßnahmen unzureichend sein können, um das Auftreten ungewollter Abbauprozesse zufriedenstellend zu vermeiden.

Der vorliegenden Erfindung liegt daher die Aufgabe zugrunde, pharmazeutische Zubereitungen zur Verfügung zu stellen, die eine erhöhte Stabilität der darin enthaltenden Wirkstoffe oder deren Salze zu ermöglichen. Das gilt insbesondere für oxidationsempfindliche Wirkstoffe. Ferner bestand die Aufgabe darin, Verfahren aufzuzeigen, welche die Herstellung solcher Zubereitungen ermöglichen.

Diese Aufgabe wird durch die Zubereitung nach dem Hauptanspruch gelöst. Vorteilhafte Weiterentwicklungen sind jeweils Gegentand entsprechender Unteransprüche.

Der Erfindung liegt dabei der Gedanke zugrunde, eine pharmazeutische Zubereitung bereitzustellen, in der der Wirkstoff durch den Zusatz eines Komplexbildners stabilisiert wird. Außerdem ist in der Zubereitung mindestens ein Polymer enthalten. Sollte es tatsächlich nur ein Polymer sein, so handelt es sich dabei um ein natürliches Polymer oder ein chemisch modifiziertes natürliches Polymer (halb-synthetisches Polymer). Sollte es eine Mischung aus mindestens zwei Polymeren sein, liegt der Anteil des natürlichen oder modifizierten natürlichen Polymers an der fertigen Zubereitung höher als der relative Anteil des darin ggf. enthaltenen rein synthetischen Polymers. Das Gleiche gilt für ein halb-synthetisches Polymer oder die Summe aus einen natürlichen und halb-synthetischen Polymer, d.h. der Anteil des natürlichen Polymers bzw. der Summe aus natürlichem und halb-synthetischen Polymer ist größer als der relative Anteil des (rein)synthetischen Polymers.

Im Kontext der vorliegenden Erfindung wird unter dem Begriff "Komplexbildner" eine Substanz oder Struktur verstanden, die durch reversible Interaktion (Komplexbildung) mit dem Wirkstoff diesen im wesentlichen vor einem Abbau schützen kann. Die Komplexbildner können organisch oder anorganisch sein. Von der Stabilisierung des Wirkstoffs in der pharmazeutischen Zubereitung bzw. von einer Zubereitung mit einem stabilen oder stabilisierten Wirkstoff ist dann auszugehen, wenn die Summe der Wirkstoffabbauprodukte in der primärverpackten Zubereitung nach einer Lagerung von 6 Tage bei 60°C kleiner ist als 0,5 %, vorzugsweise 0,4%, 0,2%, besonders bevorzugt kleiner ist als 0,1% (jeweils bezogen auf die vorhandenen Gesamtmenge an Wirkstoff). Die Bestimmung der Abbauprodukte erfolgt nach den dem Fachmann bekannten Analysemethoden, wie z.B. Flüssigkeitschromatographie (HPLC).

Die stabilisierende Interaktion zwischen Komplexbildner und Wirkstoff erfolgt dabei nach dem Fachmann bekannten Prinzipien. Beispiele dafür sind reversible Einschlußverbindungen, ionische Wechselwirkungen, Wechselwirkungen aufgrund van-der-Waals-Kräften, sterische Wechselwirkungen, Wechselwirkungen aufgrund Wasserstoff- und/oder Sulfidbrücken, Adsorptionseffekten, Einbettungen, etc. mit der Wirkung, dass wenigstens die bevorzugten Angriffsorte für den Abbau des Wirkstoffs gegenüber den stabilitätsmindernden Einflüssen (z.B. Reduktion oder Oxidation) abgeschirmt bzw. blockiert sind bzw. die stabilitätsmindernden Abbaureaktionen unterbunden wird.

Besonders bevorzugte Komplexbildner sind organische oder anorganische Verbindungen, die vorzugsweise Kavitäten aufweisen, die mit einem Wirkstoff eine Einschlußverbindung ausbilden können. Beispiele solcher Komplexbildner sind die dem Fachmann grundsätzlich bekannten räumliche Strukturen ausbildende Substanzen wie Poloxamere, geeignete Cellulosen und modifizierte Cellulosen, Polysaccharide (Stärken, modifizierte Stärken), Dextrine und Cyclooligosaccharide, wie vor allem Cyclodextrine (z.B. alpha-, beta- oder gamma Cyclodextrin), Cycloaltrine, Cyclomannine, Cycloalline und Cyclofructine. Diese können jeweils auch als Derivate, zum Beispiel als Ester oder Ether verwendet werden (z.B. Methyl-Cyclodextrin, Hydroxy-Propyl-Cyclodextrin, Sulfobutyliertes oder acetyliertes Cyclodextrin). Ebenso sind Mischungen aus verschiedenen Cyclooligosacchariden oder deren Derivate möglich. Nachfolgend wird, sofern nicht anders deutlich gemacht, zusammenfassend von Cyclosacchariden gesprochen.

Weitere bevorzugte Komplexbildner sind solche, die z.B. über Ladungen mit den Wirkstoffen interagieren können. Dies sind prinzipiell alle die, die entsprechende zum Wirkstoff inverse Gruppen aufweisen. Ist der Wirkstoff aus der Gruppe der Amine, sollte der Komplexbildner eine saure Gruppe aufweisen. Andere typische funktionelle Gruppen derartiger Komplexbildner können z.B. sein: Sulfonate, quartäre Ammoniumgruppen, Carbonate, tertiäre Ammoniumgruppen, Succinate, Acetate, Phthalate, Carbonsäuren etc., z.B. Polyacrylsäure, Divinylstyrolbenzolsulfonat, Carrageene, Alginate, Chitosan, Carboxymethylstärke, Carboxymethylcellulose, Aminoalkyl-Methacrylat-Copolymer, Methacrylsäure-Methylmethacrylat-Copolymer, Celluloseacetatphthalat, Hydroxypropylmethylcellulosephthalat, Schellacksuccinat, Polyvinylpyrrolidone.

Die Komplexbildner, vorzugsweise die Cyclooligosaccharide, insbesondere die Cyclodextrine, können in relativen Anteilen von 5 bis 70% (w/w), vorzugsweise von 10 bis 50% (w/w) in der fertigen Zubereitung vorliegen. Besonders vorteilhaft sind Mengen, die bezogen auf die Interaktion mit dem Wirkstoff ein gleiches molares Verhältnis aufweisen.

Der Begriff "natürliches Polymer" wird im Rahmen der vorliegenden Erfindung als ein nicht durch chemische Synthese gewonnenes Polymer verstanden. Beispiele für natürliche Polymer sind Cellulosen und Stärken. Unter einem halb-synthetischen Polymer wird ein chemisch modifiziertes "natürliches Polymer", z.B. Hydroxypropylcellulose verstanden.

Für die Herstellung der Polymermatrix der erfindungsgemäßen Zubereitung werden insbesondere folgende natürlichen oder halbs-synthetischen Polymere bevorzugt: Cellulose-Ether, insbesondere Ethylcellulose, Propylcellulose, Carboxymethylcellulose, Hydroxypropylcellulose, Hydroxypropylmethylcellulose, Gemische von CelluloseEthern, sowie Celluloseacetat, Alginate, Pectine, Gelatine, alle natürlichen und modifizierten Stärken, und natürliche Gummen.

In einer Ausführungsform der Erfindung beträgt der relative Anteil der natürlichen bzw. halbsynthetischen Polymere mindestens 5 bis 90 Gew.-%, besonders bevorzugt mindestens 15-60 Gew.-%, besonders bevorzugt mindestens 30 bis 50 Gew.-%.

Davon zu unterscheiden sind "synthetische Polymere". Dieser Begriff wird synonym mit "rein-synthetischen Polymeren" gebraucht. Diese werden als aus chemischer Synthese/Polymerisation gewonnene Polymere definiert. Beispiele dafür sind Polyvinylalkohole, Polyethylenglycole, Polyvinylpyrrolidone etc. Die relativen Anteile liegen vorteilhafterweise bei unter 25 Gew.-%, bevorzugt unter 10 Gew.-%. Weiterhin bevorzugt sind Anteile von unter 5Gew.-%, oder unter 2 Gew.-%.

Die Stabilität des Wirkstoffs kann noch weiter verbessert werden, indem die Peroxidzahl der Zubereitung bei der Herstellung auf einen Wert von höchstens 40, vorzugsweise höchstens 15, insbesondere von höchstens 5 eingestellt wird.

Die Peroxidzahl ist ein Mass für den Gehalt an Peroxiden; sie gibt die Menge an MilliÄquivalenten aktiven Sauerstoffs pro kg einer Substanz an. Bei einer Begrenzung der Peroxidzahl auf einen Höchstwert von 40, vorzugsweise von 15 und insbesondere von 5, sind die erfindungsgemäßen Zubereitungen im wesentlichen frei von aktivem Sauerstoff.

Unter "aktivem Sauerstoff' wird Sauerstoff verstanden, der eine Oxidationsstufe von grösser als -2 aufweist. Insbesondere umfasst dieser Begriff molekularen Sauerstoff sowie Peroxide der allgemeinen Struktur R-O-O-R', worin R und R'H-Atome sind, oder R ein Alkylrest und R' ein H-Atom ist, oder R und R'Alkylreste sind, wobei R und R' identisch oder verschieden sein können.

Es hat sich gezeigt, dass eine akzeptable, vorzugsweise über Monate andauernde Lagerstabilität nur erreicht werden kann, wenn der relative Anteil des aktiven Sauerstoffs den Wert von 2 % (w/w) nicht übersteigt. Beispielsweise kann eine wirkstoffhaltige filmförmige Zubereitung ("wafer") 200 mg eines Wirkstoffs mit einem Molekulargewicht von 250 Dalton enthalten, welches 0,8 mmol Wirkstoff entspricht. Der Anteil des aktiven Sauerstoffs sollte in diesem Fall nicht mehr als 2 % dieses Wertes betragen, d. h. ein Gehalt von 0,016 mmol aktiven Sauerstoffs darf nicht überschritten werden. Dieser Wert entspricht einer Peroxidzahl von ca. 30. Bei dem genannten Wafer kann es sich beispielsweise um einen lutschbaren Wafer mit einem Flächengewicht von 500 g/m2 und einer Wirkstoffbeladung von 40 % handeln, mit einer Flächenausdehnung von 10 cm2 handeln.

Im Falle von dünneren, schnell freisetzenden Wafern mit einer niedrigeren Wirkstoffbeladung ergibt sich eine entsprechend niedrigere Obergrenze für Gehalt an aktivem Sauerstoff. Wenn der vorstehend erwähnte Wirkstoff (Molekulargewicht 250 Dalton) in einer Menge von 14 mg in einem Wafer enthalten ist, entspricht dies 0,056 mmol Wirkstoff, und folglich sollte der Gehalt an aktivem Sauerstoff den Wert von 0,001 mmol (entsprechend 2 %) nicht überschreiten. Dies entspricht einer Peroxidzahl von ca. 14. Der genannte Wafer kann beispielsweise ein Flächengewicht von 70 g/m2 und eine Wirkstoffbeladung von 20 Gew.-% aufweisen, wobei das Eigengewicht eines einzelnen Systems (Wafer) 70 mg beträgt.

Für die Bestimmung der Peroxidzahl gibt es verschiedene dem Fachmann bekannte Methoden.
(A) Am gebräuchlichsten ist die Umsetzung einer definierten Menge der zu testenden Substanz in einer Chloroform/Eisessig-Lösung mit einem Überschuss an lodid-lonen und anschliessender Rücktitration des gebildeten lods mit Natriumthiosulfat.
(B) Weniger gebräuchlich und auf wässrige Lösungen beschränkt ist die Umsetzung der zu testenden Substanz mit Titan(IV)-lonen und die photometrische Bestimmung des sich bildenden Peroxokomplexes.
(C) Besonders einfach durchzuführen ist ein semiquantitativer Peroxid-Test mit kommerziell erhältlichen Teststäbchen.

Diese besondere Ausführungsform der Erfindung beruht auf der Erkenntnis, dass die bei der Herstellung der filmförmigen Zubereitungen verwendeten Rohstoffe oder Formulierungsbestandteile im Ausgangszustand häufig relativ hohe Konzentrationen an Hydroperoxiden und Peroxiden aufweisen. Dies trifft häufig auf Polymere, Lösemittel und bestimmte Zusatzstoffe zu. Begünstigt durch die Gegenwart von Luftsauerstoff und Schwermetall-Verunreinigungen, kommt es zu Radikalkettenreaktionen, in deren Verlauf bestimmte Bindungen in Wirkstoffmolekülen angegriffen werden, z. B. C-H-Bindungen in Benzyl- oder Allylstellung, tertiäre C-H-Bindungen und C-H-Bindungen in der Nähe von Ethersauerstoffatomen. Wirkstoffmoleküle, die über solche Gruppen verfügen, sind in besonderem Masse durch Peroxid-Angriff gefährdet.

Durch diese Verminderung der Peroxidzahl in der Zubereitung lässt sich die durch Radikalkettenreaktionen verursachte Wirkstoffinstabilität zumindest zum Teil unterdrücken. Dies ist dann besonders vorteilhaft, wenn schon die verwendeten Ausgangsmaterialien bzw. Formulierungsbestandteile relativ hohe Konzentrationen an Peroxiden bzw. Hydroperoxid-Radikalen enthalten.

Grundsätzlich lässt sich die Peroxidzahl der hergestellten filmförmigen Zubereitungen nach den oben beschriebenen Methoden (A) oder (B) bestimmen. Allerdings kann es unter Umständen schwierig sein, eine ausreichende Menge der zu testenden Zubereitung in einer nicht zu grossen Menge des angegebenen Lösemittels zu lösen.

Zur Vereinfachung wird deshalb vorzugsweise so vorgegangen, dass der Peroxid-Gehalt eines jeden Formulierungsbestandteils der filmförmigen Zusammensetzung einzeln bestimmt wird (beispielsweise nach einer der oben angegebenen Methoden), und anschließend die Peroxidzahl der Zusammensetzung berechnet wird, wobei die Peroxidzahlen der einzelnen Formulierungsbestandteile entsprechend ihrem prozentualen Anteil an der Zusammensetzung gewichtet und schliesslich addiert werden; diese Summe stellt die Gesamt-Peroxidzahl der Zusammensetzung dar. Bei der Berechnung der Gesamt-Peroxidzahl ist auch der Peroxid-Gehalt der bei der Herstellung verwendeten Lösemittel zu berücksichtigen.

Die Berechnung der Gesamt-Peroxidzahl wird durch folgendes Rechenbeispiel veranschaulicht: Eine filmförmige Zusammensetzung besteht aus drei Formulie- rungsbestandteilen X, Y und Z, wobei der Anteil von X 70 Gew. -%, der Anteil von Y 20 Gew. -% und der Anteil von Z 10 Gew. -% beträgt. Für Bestandteil X wurde eine Peroxidzahl von 10 bestimmt, im Falle von Y und Z beträgt die Peroxidzahl 15 bzw. 30.

Die Gesamt-Peroxidzahl berechnet sich wie folgt: (10 x 70/100) + (15 x 20/100) + (30 x 10/100)==7+3+3=13_{.}

Wie ersichtlich, werden die Peroxidzahlen der einzelnen Bestandteile mit einem Faktor gewichtet, der ihrem prozentualen Anteil an der Zusammensetzung entspricht.

Die Stabilität des Wirkstoffs in der erfindungsgemäßen Zusammensetzung kann noch weiter verbessert werden, indem mindestens ein Antioxidans hinzugefügt wird. Möglich sind insbesondere Antioxidantien aus Gruppe, die Ascorbinsäure, Ascorbylpalmitat, Natriumsulfit, Natriumdisulfit, Natriummetabisulfit, EDTA, Citronensäure, Thioglycerol, Thioglykolsäure, Tocopherole (Vitamin E), Tocopherolacetat, Vitamin A, Propylgallat, Octylgallat, Butylhydroxyanisol und Butylhydroxytoluol umfasst.

Daneben kommen auch viele Antioxidantien in Betracht, die in der Lebensmittelindustrie eingesetzt werden. Die Konzentration dieser Stoffe beträgt vorzugsweise 0,001 bis 5 Gew. -%, besonders bevorzugt 0,01 bis 3 Gew.-%, jeweils bezogen auf die filmförmige Zusammensetzung.

Die Zubereitung kann in einer Ausführungsform als schnell zerfallender pharmazeutischer Film, insbesondere für die orale Administration, ausgebildet sein. Die Dicke dieses Films liegt vorzugsweise im Bereich von 0,01 bis 5 mm, besonders bevorzugt im Bereich von 0,05 bis 1 mm.

Als matrixbildende Polymere, welche Bestandteile der erfindungsgemässen Zubereitung sein können, kommen - ohne andere geeignete Rohstoffe auszuschliessen - vorzugsweise folgende Polymere in Betracht: Cellulosederivate wie Hydroxypropylmethylcellulose, Hydroxypropylcellulose, Natri-um-Carboxymethylcellulose (z. B. Walocel), Methylcellulose, Hydroxyethylcellulose und Hydroxypropylethylcellulose; Stärke und Stärkederivate; Gelatine (verschiedene Typen), Gummi arabicum oder Pullulan.

Als Wirkstoffe, die in den erfindungsgemässen Zubereitungen enthalten sind, kommen grundsätzlich alle pharmazeutischen Wirkstoffe in Betracht, sowie alle anderen Wirkstoffe, die geeignet sind, in physiologische Vorgänge bei Mensch oder Tier einzugreifen.

Die erfindungsgemässen Zubereitungen eignen sich insbesondere zur Verabreichung von Wirkstoffen, die aufgrund ihrer chemischen Struktur in erhöhtem Masse für oxidative Abbaureaktionen empfänglich sind. Hierzu gehören vor allem Wirkstoffmoleküle, die über mindestens eine der folgenden Teilstrukturen verfügen:
- sekundäre oder tertiäre Aminogruppen
- ungesättigte Bindungen wie z.B. C-C-Doppel- und/oder Dreifachbindungen, einzeln oder konjugiert
- C-H-Gruppen in Allylstellung
- benzylische C-H-Gruppen
- tertiäre C-H-Gruppen
- Sulfid-, Thioether-oder Sulfoxidgruppen.

Beispiele für solche Wirkstoffe sind: Steroide wie 17-beta- Estradiol, heterozyklische Verbindungen wie Dihydropyridine (z. B. Calciumantagonisten vom Dihydropyridin-Typ), Nicotin, (-) -5,6, 7, 8,-Tetrahydro-6- [propyl [2- (2-thienyl)- ethyl] amino]-I-naphthol, aromatische Verbindungen, insbesondere substituierte aromatische Verbindungen (z. B. Adrenalin, Salicylsäure und -derivate, Phenothiazine); sauerstoffempfindliche Biopolymere, Proteine, oxidationsanfällige Stoffe wie Amine, Hydroxylamine, Alkohole und Aldehyde.

Weitere instabile Wirkstoffe, die erfindungsgemäß eingesetzt werden können - ohne auf deren Verwendung eingeschränkt zu sein - sind dem Fachmann bekannt, z.B. Amlodipin, Felodipin, Nifedipin, Captopril, Corticoide, Desloratadin, Isotretinoin, Betamethason, Codein, Calcitriol, Diclofenac und/oder deren pharmazeutisch akzeptablen Salze.

In einer Ausführungsform der Erfindung handelt es sich bei dem Wirkstoff um Donepezil, insbesondere um Donepezil-Hydrochlorid.

### •(+/-)-2-[(1-Benzyl-4-piperidyl)methyl]-5,6-dimethoxy-1-indanone (hydrochloride)

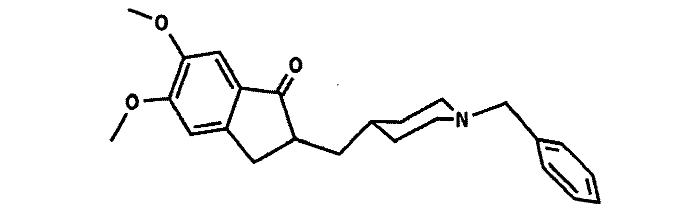

Donepezil ist dessen Salze sowie deren Herstellung sind dem Fachmann zum Beispiel aus der EP 296560 B1 bekannt. Ebenso ist dem Fachmann bekannt, dass Donepezil-Salze, insbesondere auch das Hydrochlorid in verschiedenen polymeren Formen vorliegen kann, so beispielsweise in den Formen I bis V (oder Form A-C). Besonders bevorzugt nach der Erfindung ist die Verwendung der Form I des Donepezil-Hydrochlorids.

Zur Erzielung bestimmter Wirkungen oder zur Modulation der chemischen oder physikalischen Eigenschaften kann es vorteilhaft sein, wenn die erfindungsgemäße Zubereitungen, insbesondere in Form einer filmförmigen Zubereitung, einen oder mehrere Zusatzstoffe enthalten ausgewählt aus der Gruppe der Weichmacher, Farbstoffe und Pigmente, Zerfallsförderer, Netzmittel, Quell- und Sprengmittel, resorptions- oder permeationsfördernde Substanzen, pH-Regulatoren, Füllstoffe, Fließregulierungsmittel, Geschmacks- und Aromastoffe und Süssstoffe. Hierfür geeignete pharmazeutisch verträgliche Stoffe sind dem Fachmann bekannt. Diese Zusatzstoffe können vorzugsweise in einer Gesamtkonzentration von bis zu 50 Gew. -% enthalten sein, insbesondere in einer Gesamtkonzentration von 1,0 bis 40 Gew.-%., vorzugsweise von 5 bis 30 Gew.-%.

Als Weichmacher kommen beispielsweise solche aus der Gruppe der Kohlenwasserstoffe, Alkohole (insbesondere höhere Alkohole wie Dodecanol, Undecanol, Octanol), Propylenglycol, Glycerol, Triglyceride, mehrwertige Alkohole, Carbonsäuren, Derivate von Carbonsäuren, Ether, Ester (z. B. Diethylphthalat, n-Butyladipat, CitronensäureEster) und Amine in Betracht. Weichmacher sind vorteilhafterweise in einer Gesamtkonzentration von 0,5 bis 40 Gew.-% , vorzugsweise von 1 bis 20 Gew.-% enthalten.

Als Resorptions- oder Permeationsbeschleuniger (Enhancer) eignen sich insbesondere Stoffe, die ausgewählt sind aus der Gruppe, die folgende Stoffe bzw. Stofflclassen umfasst: gesättigte oder ungesättigte Fettsäuren, Fettsäure-Ester, insbesondere Ester mit Methanol, Ethanol oder Isopropanol (z. B. Ölsäureethylester, Ölsäuremethylester, Laurinsäure- methylester, Laurinsäureethylester, Adipinsäuremethylester, Adipinsäureethylester), geradkettige oder verzweigte Fett- alkohole bzw. deren Ester, insbesondere Ester mit Essigsäure oder Milchsäure (z. B. Ethyloleat, Ethyllaurat, Ethyl- palmitat, Ethyllactat, Propyllactat, Propylpalmitat, Propyllaurat, Propyloleat), mehrwertige aliphatische Alkohole oder Polyethylenglykole, Sorbitanfettsäureester und deren durch Ethoxylierung erhältlichen Derivate, Fettalkohol- ethoxylate, Polyoxyethylenfettsäureester; Laurinsäurediet- hanolamid, Ölsäurediethanolamid, Kokosfettsäurediethanola- mid, D-alpha-Tocopherol, Laurinsäurehexylester, 2-Octyl- dodecanol, Dexpanthenol, lsopropylidenglycerol, Transcutol (= Diethylenglycol-monoethylether), DEET (= N, N-Diethylm- Toluolamid), Solketal, Ethanol, 1, 2-Propandiol oder andere kurzkettige Alkohole (d. h. Alkohole mit bis zu 6 C-Atomen), sowie Menthol und andere ätherische Öle oder Bestand- teile ätherischer Öle. Um den Wirkstoff-Flux zu optimieren, können auch Kombinationen zweier oder mehrerer Enhancer eingesetzt werden.

Die erfindungsgemässen Zubereitungen können als orale Darreichungsformen verwendet werden, welche die Freisetzung und/oder Resorption von pharmazeutischen Wirkstoffen bereits im Mund (bukkale Resorption) und/oder im Gastrointestinaltrakt ermöglichen. Beispielsweise kann im Falle von lutschbaren filmförmigen Zubereitungen die beim Lutschen entstehende wirkstoffhaltige Lösung oder Suspension geschluckt und anschliesend im Gastrointestinaltrakt resorbiert werden. Als lutschbare wirkstoffhaltige Systeme werden bevorzugt relativ dicke Filme verwendet, vorzugsweise mit einer Dicke von bis zu 5 mm, insbesondere von 0,5 bis 5 mm.

Die Erfindung umfasst aber auch insbesondere solche orale filmförmige Arzneiformen, die zum Schlucken bestimmt sind und bei denen die Wirkstofffreisetzung im wesentlichen erst im Gastrointestinaltrakt einsetzt. Dies schliesst auch solche filmförmige wirkstoffhaltige System mit ein, die nach oraler Verabreichung zunächst in der Mundhöhle innerhalb weniger Minuten zerfallen, die dabei entstehende wirkstoffhaltige Lösung oder Suspension geschluckt und anschliessend im Gastrointestinaltrakt resorbiert werden. Ein derart schnell zerfallender Film ist insbesondere für Donepezil oder dessen pharmazeutisch akzeptablen Salze geeignet.

Die vorliegende Erfindung betrifft des weiteren Herstellungsverfahren, mittels derer filmförmige Zubereitungen der vorstehend genannten Art erhalten werden können.

Dem Fachmann sind geeignete Methoden zur Ausbildung des Komplexes zwischen Komlexbildner und Wirkstoff bekannt. Sie kann zum Beispiel vor der Einbringung des Wirkstoffs in den Gesamtansatz erfolgen, aber auch - z.B. bei ionischer Wechselwirkung zwischen Wirkstoff und Komplexbildner - *in-situ* erfolgen. In diesem Fall werden der Wirkstoff und der Komplexbildner in einem geeigneten Lösungsmittel zusammengebracht, so ggf. in einem wässrigen System. Der pH-Werts kann entsprechend angeaßt werden.

Im Falle der Einschlussverbindungen mit einem Cyclosaccahrid, insbesondere mit einem Cyclodextrin, kann der Wirkstoff mit dem Komplexbildner vorab trocken verrieben werden und/oder dem Granulieren ähnlich mit Wasser vorverrieben werden. Bei Adsorptionswechselwirkungen wird z.B. eine Wirkstofflösung zunächst auf das Substrat aufgebracht und anschließend das Lösungsmittel durch Trocknen entfernt. Wird eine Herstellart aus der Schmelze gewählt (z.B. Extrusion), kann auf die Vorbehandlung des Wirkstoffes mit dem Komplexbildner zumeist verzichtet werden.

In einer vorteilhaften Ausgestaltung dieses Verfahrens kann außerdem in einem ersten Schritt die Peroxidzahl eines jeden der für die Herstellung der Zubereitung gemäss Rezeptur vorgesehenen Formulierungsbestandteils (einschließlich der verwendeten Lösemittel) bestimmt wird.

Anschliessend können in einem weiteren Schritt die Formulierungsbestandteile in der Weise so ausgewählt werden, dass die Sümme der Peroxidzahlen der einzelnen Formulierungsbestandteile höchstens 40 beträgt, wobei die Peroxidzahl eines jeden Formulierungsbestandteils entsprechend dem prozentualen Anteil dieses Bestandteils an der Zubereitung gewichtet wird.

Aus diesen so ausgewählten Formulierungsbestandteilen kann eine Lösung, Dispersion oder Schmelze hergestellt werden, welche den/die Wirkstoff (e) enthält.

Diese Lösung, Dispersion oder Schmelze kann anschließend durch Rakel-, Walzenauftrags-, Sprüh-oder Extrusionsverfahren auf eine inerte Unterlage beschichtet werden. Es folgt dann eine Trocknung und Abkühlung. Dadurch bildet sich die Filmschicht aus.

Falls sich bei der Bestimmung der Peroxidzahlen der einzelnen Komponenten herausstellt, dass ein zu hoher Peroxidgehalt vorliegt, so kann entweder ein Ersatzstoff für diese Komponente (z. B. ein Rohstoff eines anderen Herstellers) ausgewählt werden, der möglicherweise eine niedrigere Peroxidzahl aufweist, oder der betreffende Formulierungsbestandteil wird einer Behandlung unterworfen, welche geeignet ist, den Peroxidgehalt zu vermindern.

Dazu kommt wahlweise auch eine Behandlung mit einem oder mehreren Reduktionsmitteln in Betracht, beispielsweise mit einem anorganischen Sulfit oder Hydrogensulfit, vorzugsweise mit Natriumsulfit oder Natriumhydrogensulfit, jeweils in wässriger Lösung (z. B. 5 bis 30 Gew. -%). Der betreffende Formulierungsbestandteil kann dabei in einer alkoholischen Lösung, vorzugsweise in methanolischer oder ethanolischer Lösung, mit der genannten wässrigen Lösung des Reduktionsmittels versetzt werden Durch diese Behandlung werden die anwesenden Peroxide in schneller Reaktion problemlos zerstört.

Abhängig von den Lösungseigenschaften des zu behandelnden Bestandteils kann dieser auch in einem wässrigen Lösungsmittel gelöst werden, oder in einem Alkohol-Wasser-Gemisch.

Falls es sich bei dem zu behandelnden Formulierungsbestandteil oder Hilfsstoff um eine Flüssigkeit handelt (z. B. Lösemittel), kann die Behandlung in der Weise erfolgen, dass diese Flüssigkeit direkt mit einer wässrigen Lösung des Re duktionsmittels (z. B. Natriumsulfit) versetzt wird.

Die Verwendung von Natriumsulfit bzw. Natriumhydrogensulfit ist besonders vorteilhaft, da es sich hierbei um pharmazeutisch gut bekannte Hilfsstoffe handelt, so dass eine spätere Abtrennung nicht erforderlich ist.

Falls es zur Ausfällung von Reaktionsprodukten kommt, können diese durch Zentrifugation, Sedimentation oder Filtration abgetrennt werden.

Nach dieser Behandlung sind die Materialien im wesentlichen frei von Peroxiden und können selbst bei vorheriger erheblicher Belastung bedenkenlos eingesetzt werden.

Eine zusätzliche Verbesserung der Stabilität kann durch den Zusatz von Anti oxidantien erreicht werden, die die Bildung neuer Peroxide während der Lagerung der Systeme unterdrücken bzw. verlangsamen. Geeignete Antioxidantien sind auch hier z.B. EDTA, BHT, BHA, Phenolderivate, Anisolderivate, Kresolderivate, Vitamin A, Vitamin A-Derivate, Vitamin E, Vitamin E-Derivate einzeln oder in Kombination.

Die Erfindung und ihre vorteilhaften Eigenschaften werden durch die nachfolgenden Beispiele näher erläutert.

**Beispiele:**

| **Inhattsstoff** | **Masse [mg/6 cm²]** | **Masse [mg/6 cm²]** | **Masse [mg/6 cm²]** | **Masse [mg/6 cm²]** | **Masse [mg/6 cm²]** | **Masse [mg/6 cm²]** | **Masse [mg/6 cm²]** | **Masse [mg/6 cm²]** |
|---|---|---|---|---|---|---|---|---|
| Donepezil HCl | 10 | 10 | 10 | 10 | 10 | 10 | 10 | 10 |
| β-Cyclodextrin | 22,25 | 22,25 | 22,25 | 22,25 | 22,25 | 22,25 | 22,25 | 22,25 |
| HPMC (Methocel E 15 LV) | 15,53 | 15,53 | 15,53 | 15,53 | 15,53 | 15,53 | 15,53 | 15,53 |
| Xanthan | 0,3 | 0,3 | 0,3 | 0,3 | 0,3 | 0,3 | 0,3 | 0,3 |
| BHT | 0,06 | 0,06 | 0,06 | 0,06 | 0,06 | 0,06 | 0,06 | 0,06 |
| Isopropylmyristat | 1,035 | 1,035 | 1,035 | --- | 1,035 | 1,035 | --- | 0,7 |
| Citronensäure | 5 | --- | --- | --- | --- | --- | --- | --- |
| Propylenglykol | --- | --- | --- | --- | 3,5 | --- | --- | --- |
| EDTA | --- | 1 | 1 | 1 | 1 | 1 | 1 | 1 |
| **Glycerin** | -- | --- | 3,5 | 3,5 | --- | --- | --- | --- |
| Triacetin | --- | --- | --- | --- | --- | --- | --- | --- |
| Dibutylsebacat | --- | --- | --- | --- | --- | 2,8 | 2,8 | --- |
| Σ VU (6d/60°C) | 0,19 | --- | --- | --- | --- | --- | --- | --- |
| Donepezil HCl | 10 | 10 | 10 | 10 | 10 | 10 | 10 | |
| β-Cyclodextrin | 22,25 | 22,25 | 22,25 | 22,25 | 22,25 | 22,25 | 22,25 | |
| HPMC (Methocel E 15 LV) | 15,53 | 15,53 | 15,53 | 15,53 | 15,53 | 15,53 | 15,53 | |
| Xanthan | 0,3 | 0,3 | 0,3 | 0,3 | 0,3 | 0,3 | 0,3 | |
| BHT | 0,06 | 0,06 | 0,06 | 0,06 | 0,06 | 0,06 | 0,06 | |
| Isopropylmyristat | --- | --- | --- | 1,035 | 1,035 | 1,035 | 1,035 | |
| Citronensäure | --- | --- | --- | --- | --- | --- | --- | |
| EDTA | 1 | 1 | 1 | 1 | 1 | 1 | 1 | |
| Tween 80 | 3,5 | --- | --- | 3,5 | --- | --- | --- | |
| Cremophor RH 40 | --- | --- | --- | --- | 3,5 | --- | --- | |
| Macrogol-8-stearat | --- | 3,5 | --- | --- | --- | 3,5 | --- | |
| Lutrol F 68 | --- | --- | 3,5 | --- | --- | --- | 3,5 | |
| Σ VU (6d/60°C) | --- | --- | --- | 0,09 | --- | --- | --- | |
| Donepezil HCl | 10 | 10 | 10 | 10 | 10 | 10 | 10 | |
| β-Cyclodextrin | 22,25 | 22,25 | 22,25 | 22,25 | 22,25 | 22,25 | 22,25 | |
| HPMC (Methocel E 15 LV) | 15,53 | 15,53 | 15,53 | 15,53 | --- | --- | 15,53 | |
| HPMC (Methocel E 5 LV) | --- | --- | --- | --- | 15,53 | 20 | 15,53 | |
| Xanthan | 0,3 | 0,3 | --- | --- | --- | --- | --- | |
| BHT | 0,06 | --- | 0,06 | 0,06 | 0,06 | 0,06 | 0,06 | |
| EDTA | --- | 1 | 1 | 1 | 1 | 1 | 1 | |
| Glycerin anhydr. | 3,5 | 3,5 | 7 | 7 | 7 | 9,3 | 9,3 | |
| Acesulfam K | --- | --- | --- | 1,5 | 1,5 | 1,5 | 1,5 | |
| Aroma Peppermint | --- | --- | --- | 4 | 4 | 4 | 4 | |
| Titandioxid | --- | --- | --- | 3 | --- | --- | --- | |
| Σ VU (6d/60°C) | --- | --- | --- | | | | | |

### Stabilitätstest

Filmförmige Zubereitungen mit den in den Beispielen angegebenen Zusammensetzungen wurden einem Stabilitäts-Test unterzogen. Hierbei wurden die Filme bei 60°C primärverpackt gelagert und nach 6 Tagen die Menge an Wirkstoffabbauprodukten per HPLC bestimmt. Die Ergebnisse sind als Σ VU in den obigen Tabellen angeführt, wobei die Prozentwerte den Gehalt an Abbauprodukten, bezogen auf den Gehalt an aktiver Substanz (Wirkstoff) angeben.

## Patentansprüche

1. Pharmazeutische Zubereitung, insbesondere eine filmförmige Zubereitung, enthaltend wenigstens einen pharmazeutischen Wirkstoff oder dessen Salz **gekennzeichnet durch**
- mindestens einen Komplexbildner zur Stabilisierung des Wirkstoffs oder dessen Salzes sowie
- mindestens eins oder mehrere Polymere ausgewählt aus der Gruppe der natürlichen oder halb-synthetischen oder synthetischen hydrophilen Polymere,
wobei der Anteil der natürlichen und halb-synthetischen Polymere größer ist als der Anteil der synthetischen Polymere.

2. Zubereitung nach Anspruch 1, **dadurch gekennzeichnet, dass** das natürliche Polymer in einem prozentualen (w/w) Anteil an der Gesamtzubereitung von 5 bis 90Gew.-%, vorzugsweise von 15 bis 60 Gew.-% und der Komplexbildner in einem Anteil von 0,1 bis 70 Gew.-%, vorzugsweise 10 bis 50 Gew.% vorliegt.

3. Zubereitung nach Anspruch 1 oder 2, **dadurch gekennzeichnet, dass** die Zubereitung eine PeroxidZahl aufweist, die höchstens 40 beträgt.

4. Zubereitung nach einem der vorangehenden Ansprüche, **dadurch gekennzeichnet, dass** sie eine Peroxid-Zahl aufweist, die höchstens 15, vorzugsweise höchstens 5 beträgt.

5. Zubereitung nach einem der vorangehenden Ansprüche, **dadurch gekennzeichnet, dass** sie im wesentlichen frei ist von aktivem Sauerstoff, wobei sich der Begriff "aktiver Sauerstoff' auf molekularen Sauerstoff sowie auf sauerstoffhaltige Verbindungen bezieht, in denen Sauerstoff eine höhere Oxidations- stufe als -2 aufweist, insbesondere Peroxide mit der allgemeinen Struktur R-O-O-R', worin R und R' aus der Gruppe ausgewählt sind, die aus Alkylresten und Wasserstoff besteht, und wobei R und R' gleich oder verschieden sein können.

6. Zubereitung nach einem der vorangehenden Ansprüche, **dadurch gekennzeichnet, dass** sie mindestens ein Antioxidans enthält, vorzugsweise ausgewählt aus der Gruppe, die Ascorbinsäure, Ascorbylpalmitat, Natriumsulfit, Natriumdisulfit, Natriummetabisulfit, Tocopherole (Vitamin E), Tocopherolacetat, Thioglycerol, Thioglykolsäure, Vitamin A, Propylgallat, Octylgallat, EDTA, Citronensäure, Butylhydroxyanisol und Butylhydroxytoluol umfasst.

7. Zubereitung nach Anspruch 6, **dadurch gekennzeichnet, dass** die Konzentration des Antioxidans/der Antioxidantien 0,001 bis 5 Gew. -%, vorzugsweise 0, 01 bis 3 Gew. -% beträgt.

8. Zubereitung nach einem der vorangehenden Ansprüche, **dadurch gekennzeichnet, dass** das natürliche Polymer ausgewählt ist aus der Gruppe, welche Cellulose-Ether, insbesondere Ethylcellulose, Propylcellulose, Carboxymethylcellulose (CMC), Hydroxypropylcellulose (HPC), Hydroxypropylmethylcellulose (HPMC), Gemischen von CelluloseEthern, sowie Celluloseacetat, Alginate, Pectine, Gelatine, Stärke (natürliche und modifizierte), und natürliche Gummen umfasst.

9. Zubereitung nach einem der vorherigen Ansprüche, **dadurch gekennzeichnet, dass** der Komplexbildner ausgewählt ist aus der Gruppe, welche alle natürlichen und chemisch modifizierten Cyclodextrine und/oder Dextrine; Poloxamere; strukturausbildende Cellulosen, Stärken und Polysaccharide; Oligomere und/oder Polymere mit funktionelle Gruppen wie: Sulfonate, quartäre Ammoniumgruppen, Carbonate, tertiäre Ammoniumgruppen, Succinate, Acetate, Phthalate, Carbonsäuren, saure Polyole etc., z.B. Polyacrylsäure, Divinylstyrolbenzolsulfonat, Carrageene, Alginate, Chitosan, Carboxymethylstärke, Carboxymethylcellulose, Aminoalkyl-Methacrylat-Copolymere, Methacrylsäure-Methylmethacrylat-Copolymere, Celluloseacetatphthalat, Hydroxypropylmethylcellulosephthalat, Schellacksuccinat, Polyvinylpyrrolidone ,einzeln oder in Kombination umfasst.

10. Verwendung einer Zubereitung nach einem der vorangehenden Ansprüche zur Verabreichung von Arzneimittelwirkstoffen, vorzugsweise zur Applikation in der Mundhöhle.
